# EUROPEAN PATENT SPECIFICATION

(11) **EP 3 524 166 B1**
(45) Date of publication and mention of the grant of the patent: **31.12.2025**
(21) Application number: 18188544.3
(22) Date of filing: 10.08.2018
(51) Int. Cl.: A61B 8/08, A61B 8/12, A61B 10/00, G16H 50/30

(54) **ULTRASOUND IMAGING APPARATUS AND CONTROL METHOD FOR THE SAME**
VORRICHTUNG ZUR MEDIZINISCHEN BILDGEBUNG UND STEUERUNGSVERFAHREN DAFÜR
APPAREIL D'IMAGERIE PAR ULTRASONS ET SON PROCÉDÉ DE COMMANDE

(30) Priority: 13.02.2018 KR 20180017773
(43) Date of publication of application: 14.08.2019
(73) Proprietor: Samsung Medison Co., Ltd., Hongcheon-gun, Gangwon-do, 25108 (KR)
(72) Inventor: Lee, Bong Heon, Seoul (KR); Shin, Dong Kuk, Gyeonggi-do (KR)
(74) Representative: Arnold & Siedsma

(56) References cited:
- HIROSHI TSUDA ET AL: "Measurement of endometrial thickness in premenopausal women in office gynecology", REPRODUCTIVE MEDICINE AND BIOLOGY, vol. 17, no. 1, 16 September 2017 (2017-09-16), Tokyo, pages 29 - 35, XP055569923, ISSN: 1445-5781, DOI: 10.1002/rmb2.12062
- CLAUDIA T. SADRO: "Imaging the Endometrium: A Pictorial Essay", CANADIAN ASSOCIATION OF RADIOLOGISTS JOURNAL, vol. 67, no. 3, 1 August 2016 (2016-08-01), CA, pages 254 - 262, XP055569927, ISSN: 0846-5371, DOI: 10.1016/j.carj.2015.09.012
- K M NALABOFF ET AL: "Imaging the Endometrium: Disease and Normal Variants", RADIOGRAPHICS, vol. 21, no. 6, 1 November 2001 (2001-11-01), Unites States, pages 1409 - 1424, XP055569931, DOI: 10.1148/radiographics.21.6.g01nv211409
- SAKAMOTO C ET AL: "The echogenic endometrium and alterations during menstrual cycle", INTERNATIONAL JOURNAL OF GYNECOLOGY AND OBSTETRICS, vol. 20, no. 4, 1 August 1982 (1982-08-01), pages 255 - 259, XP026248222, ISSN: 0020-7292, [retrieved on 19820801], DOI: 10.1016/0020-7292(82)90051-0
- PATRICIA M. MORGAN ET AL: "Ultrasonographic Assessment of the Endometrium in Rhesus Monkeys during the Normal Menstrual Cycle", BIOLOGY OF REPRODUCTION, vol. 36, no. 2, 1 March 1987 (1987-03-01), US, pages 463 - 469, XP055569899, ISSN: 0006-3363, DOI: 10.1095/biolreprod36.2.463
- NICHOLAS J. RAINE-FENNING ET AL: "Defining endometrial growth during the menstrual cycle with three-dimensional ultrasound", BJOG: AN INTERNATIONAL JOURNAL OF OBSTETRICS AND GYNAECOLOGY, vol. 111, no. 9, 1 September 2004 (2004-09-01), GB, pages 944 - 949, XP055406857, ISSN: 1470-0328, DOI: 10.1111/j.1471-0528.2004.00214.x

## Description

### BACKGROUND

### 1. Field

Embodiments of the present disclosure relate to an ultrasound imaging apparatus configured to generate an image of the inside of an object by using ultrasonic waves and a control method for the same.

### 2. Description of Related Art.

An ultrasound imaging apparatus may send ultrasound signals, which are generated by a transducer of a probe, to a target part inside an object from a surface of the object, and receive information related to reflected ultrasound signals (ultrasound eco signal), thereby obtaining an internal image of the object.

In comparison with an X-ray apparatus, the ultrasound imaging apparatus is safe because there is no risk of radiation exposure and the ultrasound imaging apparatus is capable of displaying an image in real time. In addition, in comparison with Magnetic Resonance Image (MRI), the ultrasound imaging apparatus is inexpensive and portable and thus the ultrasound imaging apparatus is widely used in medical examination.

The article by Hiroshi Tsuda et al., "Measurement of endometrial thickness in premenopausal women in office gynecology", Reprod. Med. Biol., 2018, vol. 17 , pages 29-35, discloses an investigation into the relationship between the phase of the menstrual cycle and the endometrial thickness (ET). Different upper limits of the ET were determined for the first half of a normal cycle and the second half of a normal cycle, respectively.

### SUMMARY

Therefore, it is an aspect of the present disclosure to provide an ultrasound imaging apparatus capable of providing a variety of information, which is needed for diagnosing a target object, to a user by using ultrasound images and a control method thereof. The invention is defined in the appended claims.

Additional aspects of the present disclosure will be set forth in part in the description which follows and, in part, will be obvious from the description, or may be learned by practice of the present disclosure.

In accordance with one aspect, an ultrasound imaging apparatus according to an embodiment of the present invention includes a processor configured to obtain an ultrasound image, a display configured to display the ultrasound image, and a controller configured to identify a menstrual cycle based on a state of an endometrial region on the ultrasound image and configured to control the display to display the identified menstrual cycle.

The controller compares the image of the endometrial region with a pre-stored image, and identifies the menstrual cycle based on a result of the comparison.

The ultrasound imaging apparatus may further include an input configured to receive at least one of a user confirmation command and a user modification command related to the identified menstrual cycle, wherein when the confirmation command is input, the controller controls the display to display the identified menstrual cycle and when the modification command is input, the controller modifies the identified menstrual cycle and controls the display to display the modified menstrual cycle.

The controller may measure a diagnostic parameter by using the ultrasound echo signal and identifies whether the measured diagnostic parameter is normal, based on the identified menstrual cycle.

The diagnostic parameter may comprise at least one of a length, a height, a width, an area, and a volume of the uterus.

The controller may identify that the diagnostic parameter is not normal when the measured diagnostic parameter is not within a range that is pre-determined according to the identified menstrual cycle.

When the measured diagnostic parameter is not normal, the controller may control the display so that the display notifies the user that there is an abnormality.

The controller may control the display so that the display notifies the user that there is an abnormality by using at least one of a color marker and a warning message.

The ultrasound imaging apparatus may further comprise an input configured to receive at least one piece of information related to whether an object has a birth experience and information related to whether an object experiences menopause, wherein the controller measures the size of the uterus by using the ultrasound echo signal, and identifies whether the measured size of the uterus is normal, based on the input information.

When the measured size of the uterus is not within a reference range related to the input information, the controller may identify that the measured size of the uterus is not normal and the controller controls the display so that the display notifies the user that there is an abnormality.

In accordance with another aspect of the present invention, a control method of an ultrasound imaging apparatus comprises obtaining an ultrasound image, displaying the ultrasound image; identifying a menstrual cycle based on a state of an endometrial region on the ultrasound image, and displaying the identified menstrual cycle.

The identification of the menstrual cycle comprises comparing an image of the endometrial region with a pre-stored image, and identifying the menstrual cycle based on a result of the comparison.

The control method of the ultrasound imaging apparatus may further comprises receiving at least one of a user confirmation command and a user modification command related to the identified menstrual cycle, wherein the display of the identified menstrual cycle comprises when the confirmation command is input, displaying the identified menstrual cycle and when the modification command is input, modifying the identified menstrual cycle and displaying the modified menstrual cycle.

### BRIEF DESCRIPTION OF THE DRAWINGS

These and/or other aspects of the disclosure will become apparent and more readily appreciated from the following description of embodiments, taken in conjunction with the accompanying drawings of which:
FIG. 1 is a view illustrating an exterior of an ultrasound imaging apparatus according to an embodiment.
FIG. 2 is a control block diagram illustrating the ultrasound imaging apparatus according to an embodiment.
FIG. 3 is a view illustrating a process of measuring an endometrium of an object according to an embodiment.
FIGS. 4A to 4C are views illustrating an operation for identifying the menstrual cycle by the ultrasound imaging apparatus according to an embodiment outside the scope of the claims.
FIGS. 5A to 5C are views illustrating an example of a notification screen displayed on the ultrasound imaging apparatus according to an embodiment.
FIGS. 6A and 6B are views illustrating an operation of measuring the size of the uterus and identifying whether the measured size of the uterus is normal, by the ultrasound imaging apparatus according to an embodiment.
FIG. 7 is a flowchart illustrating a control method of the ultrasound imaging apparatus according an embodiment that is outside the scope of the claims.
FIG. 8 is a flowchart illustrating a control method of the ultrasound imaging apparatus according an embodiment that is outside the scope of the claims.

### DETAILED DESCRIPTION

In the following description, like reference numerals refer to like elements throughout the specification. Well-known functions or constructions are not described in detail since they would obscure the one or more exemplar embodiments with unnecessary detail. Terms such as "unit", "module", "member", and "block" may be embodied as hardware or software. According to embodiments, a plurality of "unit", "module", "member", and "block" may be implemented as a single component or a single "unit", "module", "member", and "block" may include a plurality of components.

It will be understood that when an element is referred to as being "connected" another element, it can be directly or indirectly connected to the other element, wherein the indirect connection includes "connection via a wireless communication network".

Also, when a part "includes" or "comprises" an element, unless there is a particular description contrary thereto, the part may further include other elements, not excluding the other elements.

As used herein, the singular forms "a," "an" and "the" are intended to include the plural forms as well, unless the context clearly indicates otherwise.

Reference will now be made in detail to embodiments of the present disclosure, examples of which are illustrated in the accompanying drawings.

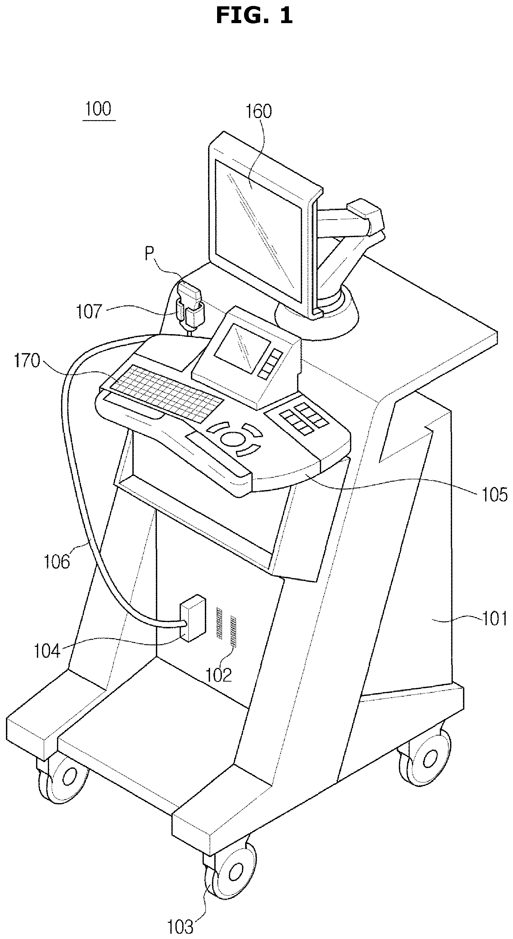

FIG. 1 is a view illustrating an exterior of an ultrasound imaging apparatus according to an embodiment.

As illustrated in FIG. 1, an ultrasound imaging apparatus 100 according to an embodiment may include a ultrasonic probe (P) transmitting ultrasonic waves to an object and receiving ultrasound echo signals from the object to convert the ultrasound echo signal into an electrical signal, a main body 101, an input 105 and a display 150.

The ultrasonic probe P corresponding to a part in contact with the surface of the object or a part inserted into a body of the object, may transmit and receive ultrasonic waves. Particularly, the ultrasonic probe (P) may transmit ultrasonic waves to the inside of the object according to a transmission signal provided from the main body 101, receive echo ultrasonic waves reflected from a specific part in the object, and transmit the echo ultrasonic waves to the main body 101.

The ultrasonic probe (P) may receive a variety of signals needed for the control of the ultrasonic probe (P) by being connected to the main body 101 via a cable 106. In addition, the ultrasonic probe (P) may transmit an analog signal or a digital signal corresponding to the ultrasonic echo signal received by the probe P, to the main body 101.

To this end, one or more female connectors 102 may be provided in one side of the main body 101. A male connector 104 provided at one end of the cable 106 may be physically coupled to the female connector 102.

However, embodiments of the ultrasonic probe (P) is not limited thereto, and the ultrasonic probe (P) may be wirelessly connected the main body 101. In this case, the ultrasonic probe (P) may be implemented as a wireless probe and thus the ultrasonic probe (P) may be configured to transmit and receive a signal via a network provided between the ultrasonic probe (P) and the main body (M). In addition, a plurality of ultrasonic probes (P) may be connected to a single main body 101.

A plurality of casters 103 moving the ultrasound imaging apparatus 100 may be provided under the main body 101. A user can fix or move the ultrasound imaging apparatus 100 by using the plurality of casters 103. The ultrasonic imaging apparatus 100 is referred to as a cart-type ultrasonic apparatus.

An operation panel 105 may be provided on a front surface of the main body 101. On the operation panel 105, an input 160 receiving an input from a user may be disposed. By using the input 160, a user may input a command for starting diagnosis, selecting a diagnostic region, selecting a diagnosis type, and selecting an ultrasonic image mode. The ultrasonic image mode may include an Amplitude mode (A-mode), a Brightness mode (B-mode), a Doppler mode (D-mode), an Elastography mode (E-mode), and a Motion mode (M-mode).

The display 150 may be provided on the upper portion of the main body 101. The display 150 may be implemented by at least one of various display panels such as a liquid crystal display (LCD) panel, a light emitting diode (LED) panel, and an organic light emitting diode (OLED) panel.

In addition, the display 150 may be provided with two or more displays so that each display may displays different images simultaneously. For example, one display may display a 2D ultrasound image and the other display may display a 3D ultrasound image. Alternatively, one display may display a B-mode image and the other display may display a contrast agent image.

On an outer peripheral surface of the main body 101, at least one probe holder 107 may be provided to hold the ultrasonic probe (P). Therefore, when users do not use the ultrasonic probe (P), the ultrasonic probe (P) may be stored in the holder 107.

In another embodiment, the ultrasound imaging apparatus 100 may be a portable ultrasound imaging apparatus that is portable for the long distance movement, wherein the portable ultrasound imaging apparatus may be not provided with a caster 103. The portable ultrasound imaging apparatus may be implemented by a fax viewer (Viewer PACS), a smart phone, a laptop computer, a PDA, or a tablet PC, but is not limited thereto.

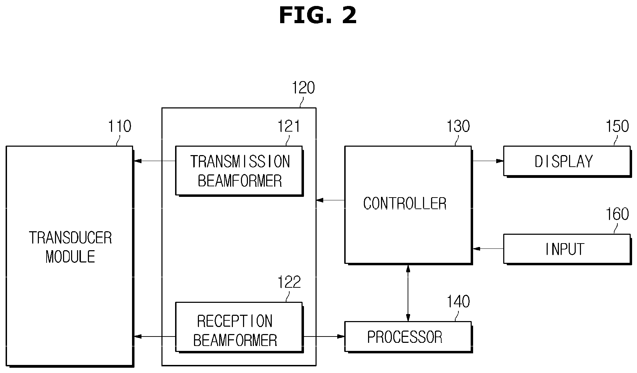

FIG. 2 is a control block diagram illustrating the ultrasound imaging apparatus according to an embodiment.

Referring to FIG. 2, the ultrasound imaging apparatus 100 according to an embodiment may include a transducer module 110 interconverting an electrical signal and an ultrasonic signal into each other, a beamformer 120 generating a transmission beam and a reception beam, a processor 140 generating an ultrasound image by using echo signals output from the beamformer 120, a controller 130 controlling the operation of internal components of the ultrasound imaging apparatus 100, the display 150, and the input 160.

The transducer module 110 may be configured to interconvert an electrical signal and an ultrasonic signal. To this end, the transducer module 110 may include a transducer array provided with a piezoelectric ultrasonic transducer element using the piezoelectric effect, and may further include a switch such as a multiplexer (MUX), configured to select a transducer element to be used transmission and reception of an ultrasonic signal.

The transducer module 110 may be provided inside the above mentioned ultrasonic probe (P). The beamformer 120 may generate a transmission beam and a reception beam and may include a transmission beamformer 121 and a reception beamformer 122.

The transmission beamformer 121 may perform transmission beamforming. The transmission beamformer 121 may generate a transmission beam by applying a time delay to the ultrasonic signal transmitted from the transducer module 110.

The generated transmission beam may be transmitted through the transducer module 110 and the transmitted ultrasonic waves may be reflected by the object and then incident to the transducer module 110. As mentioned above, when receiving echo ultrasonic waves reflected by the object, the transducer module 110 may output an echo signal corresponding to the received echo ultrasonic waves. The echo signal may be input to the reception beamformer 122.

The reception beamformer 122 may output the echo signal with a predetermined time delay, and may combine echo signals by applying a weight to the echo signals. In addition, the reception beamformer 122 may amplify the echo signal and perform a gain correction on the echo signal.

The processor 140 may generate an ultrasound image based on the echo signal output from the reception beamformer 122. For example, the processor 140 may generate at least one of an A-mode image, a B-mode image, a D-mode image, an E-mode image, and an M-mode image based on an echo signal. In addition, the processor 140 may generate a 3D ultrasound image based on the plurality of ultrasound images acquired from the echo signal.

In addition, the processor 140 may perform image processing to indicate a variety of additional information on the ultrasound image.

For this, the processor 140 may be implemented in the form of hardware such as a microprocessor, or may be implemented in the form of software executed on hardware.

The input 150 may receive a user input related to ultrasound imaging apparatus 100, and input user information. The user information may include information related to whether an object has a birth experience, and information related to whether an object experiences menopause.

The display 150 may display the generated ultrasound image and various data required for the diagnosis. In addition, the display 150 may provide information to the user by displaying a menstrual cycle identified by the controller 130 described later, and by displaying whether diagnostic data measured is normal or not. A detail description thereof will be described later.

The controller 130 may control a variety of components of the ultrasound imaging apparatus 100. The controller 130 may control the beamformer 120 so that the beamformer 120 generates a signal for acquiring a variety of data necessary for diagnosis, and the controller 130 may control the processor 140 so that the processor 140 generates an ultrasound image.

The controller 130 may measure a diagnostic parameter related to the uterus of an object on an ultrasound image generated by the processor 140. At this time, the diagnostic parameter may represent a measurement value required for diagnosis, wherein the diagnostic parameter may include a length, a height, a width, an area, and a volume.

In addition, the controller 130 may measure the diagnostic parameter for a region of interest by using the ultrasound echo signal received by the reception beamformer 122. At this time, the region of interest may be set automatically or may be input from the user through the input 160.

The controller 130 identifies the menstrual cycle of the object based on the ultrasound image generated by the processor 140, and may identify whether the measured diagnostic parameters are normal, according to the identified menstrual cycle.

The controller 130 may identify the menstrual cycle of the object with reference to the endometrial region on the ultrasound image generated by the processor 140. In an embodiment not forming part of the invention, the controller 130 may identify the menstrual cycle based on at least one of the brightness and the contrast of the endometrial region.

In accordance with the invention, controller 130 compares the ultrasound image generated by the processor 140, with at least one of a pre-stored reference image and a previously measured image. As a result of the comparison, the controller 130 may select a menstrual cycle corresponding to the image having a high matching rate, as a menstrual cycle of the object. To this, according to an embodiment, the ultrasound imaging apparatus 100 may further include a storage (not shown).

The controller 130 may identify whether the measured diagnostic parameter is normal, by depending on whether the measured diagnostic parameter is contained in a predetermined range, wherein the range is predetermined based on the identified menstrual cycle.

The controller 130 may control the display 150 so that the display 150 provides information such as information related to whether the measured diagnostic parameters is normal, as well as the ultrasonic image and the measured diagnostic parameters, in a variety of forms, to a user.

In addition, the controller 130 may store the ultrasound image, the measured diagnostic parameters, and the information related to the identified menstrual cycle in a storage (not shown).

Accordingly, a user such as a doctor can perform diagnosis of a specific disease using the ultrasound image displayed on the display 150, and more easily diagnose a specific disease, based on whether the measured diagnostic data is within a normal range.

Hereinafter, a particular operation of the controller 130 will be described in detail.

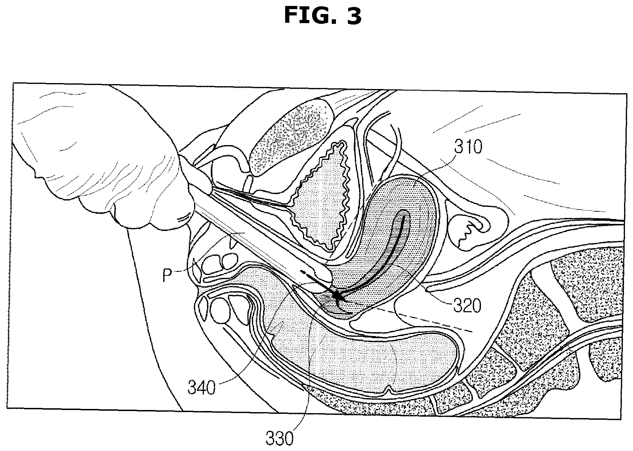

FIG. 3 is a view illustrating a process of measuring an endometrium of an object according to an embodiment.

Referring to FIG. 3, a user may insert the ultrasonic probe (P) into the body of an object and perform a diagnosis. Particularly, the user may obtain diagnostic data on a uterus 310 of the object by inserting the ultrasonic probe (P) into a cervix 330 of the object.

According to an embodiment, the controller 130 may obtain a sagittal plane or a transverse plane of the uterus 310 of the object.

The sagittal plane of the uterus 310 may be obtained when the user places a probe marker of the ultrasonic probe (P) at 12 o'clock and inserts the ultrasonic probe (P) into the upper vaginal cavity.

The transverse plane of the uterus 310 may be obtained when the user rotates the probe marker of the ultrasonic probe (P) by 90 degrees along a long axis 340 to the counterclockwise direction from a mid-sagittal plane of the uterus 310. Alternatively, the transverse plane of the uterus 310 may also be obtained when the user rotates the probe marker by 90 degrees along the long axis 340 to the clockwise direction from the mid-sagittal plane.

The controller 130 may identify a menstrual cycle based on the endometrial region 320 indicated on the ultrasound image about the uterus of the object.

The size of the endometrium may be changed by the hormonal action of the menstrual cycle. Particularly, the size of the endometrium may be increased by the hormonal action as it passes through a menstrual phase, a proliferative phase and a secretory phase.

A state of the endometrial region 320 including the change in the size of the endometrium, may be changed according to the menstrual cycle due to the hormonal action. The state of the endometrial region 320 may be checked by the ultrasound image, and the user may be supplied with data required for the diagnosis of the object by using the ultrasound image.

The controller 130 may identify the menstrual cycle of the object based on the state of the endometrial region 320 including the change in the size of the endometrium.

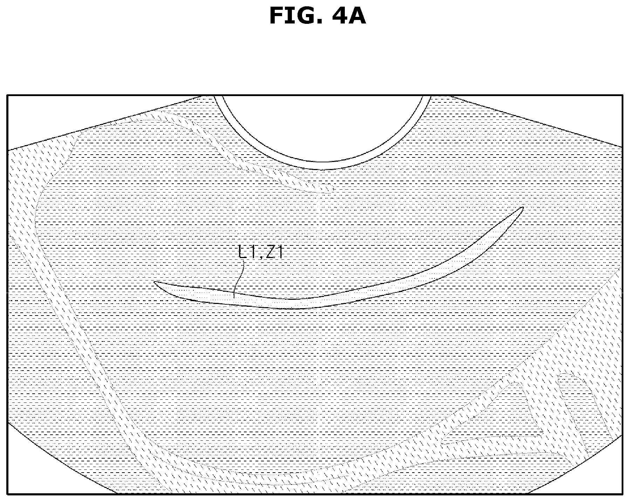

FIGS. 4A to 4C are views illustrating an operation for identifying the menstrual cycle by the ultrasound imaging apparatus according to an embodiment outside the scope of the claims.

The controller 130 may roughly divide the menstrual cycle of the object into a postmenstrual phase, a late proliferative near ovulation phase, and a post-ovulatory phase.

The controller 130 may identify the menstrual cycle corresponding to the ultrasound image during the menstrual cycle. Particularly, the controller 130 may identify the menstrual cycle corresponding to the state of the endometrial region of the ultrasound image generated by the processor 140.

FIG. 4A is a view illustrating an ultrasound image of a sagittal plane of the uterus of the object obtained by the ultrasound imaging apparatus, particularly indicating an ultrasound image of the postmenstrual phase.

As illustrated in FIG. 4A, an endometrial region (Z1) in the postmenstrual phase, may include a line (L1) having a high shade in which the contrast is higher than an adjacent region. A thickness of the endometrial region (Z1) may be relatively thin. That is, the endometrial region (Z1) in the postmenstrual phase may be identified as the form of a thin single layer.

When the endometrial region (Z1) of the ultrasound image includes the single layer (L1) having the high shade, and when the thickness of the endometrial region (Z1) is in a predetermined range (K1), the controller 130 may identify that the menstrual cycle of the object is in the postmenstrual phase. For example, the predetermined range (K1) may be equal to or greater than 3mm but, less than 8mm.

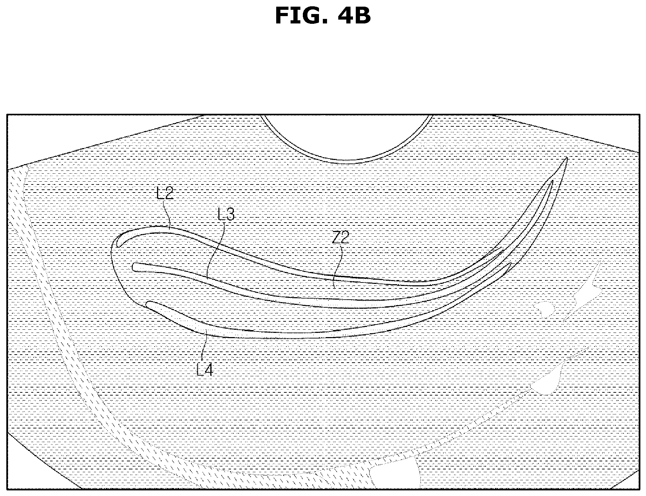

FIG. 4B is a view illustrating an ultrasound image of a sagittal plane of the uterus of the object obtained by the ultrasound imaging apparatus, particularly indicating an ultrasound image of the late proliferative near ovulation phase.

As illustrated in FIG. 4B, an endometrial region (Z2) in the late proliferative near ovulation phase, may include three lines (L2, L3 and L4) having a high shade. A thickness of the endometrial region (Z2) may be relatively thick. That is, the endometrial region (Z2) in the late proliferative near ovulation phase may be identified as the form of thick triple layer.

When the endometrial region (Z2) of the ultrasound image includes the triple layer (L2, L3 and L4) having the high shade, and when the thickness of the endometrial region (Z2) is in a predetermined range (K2), the controller 130 may identify that the menstrual cycle of the object is in the late proliferative near ovulation phase. For example, the predetermined range (K2) may be equal to or greater than 10mm, but less than 16mm.

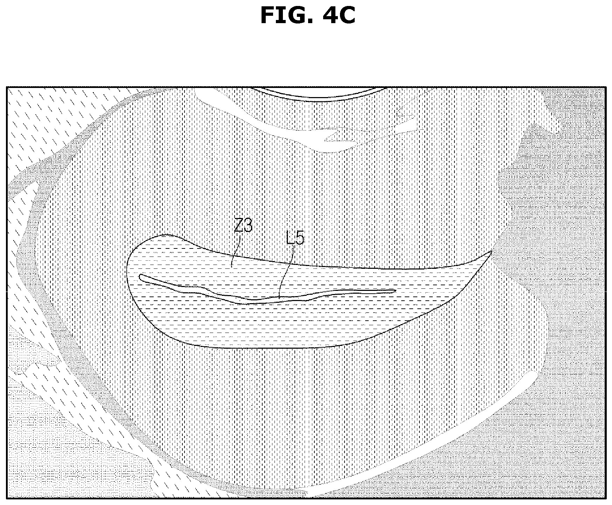

FIG. 4C is a view illustrating an ultrasound image of a sagittal plane of the uterus of the object obtained by the ultrasound imaging apparatus, particularly indicating an ultrasound image of the post-ovulatory phase.

As illustrated in FIG. 4C, an endometrial region (Z3) in the post-ovulatory phase, may include a line (L5) having a high shade. A thickness of the endometrial region (Z3) may be relatively thick. That is, the endometrial region (Z3) in the post-ovulatory phase may be identified as the form of a thick single layer.

The endometrial region (Z3) may be identified as a region having a shade distinguished from an adjacent region, and the thickness of the endometrial region (Z3) may be measured by using a height of a region having a certain shade that is distinguished from the adjacent region.

When the endometrial region (Z3) of the ultrasound image includes the single layer (L5) having the high shade, and when the thickness of the endometrial region (Z3) is in a predetermined range (K3), the controller 130 may identify that the menstrual cycle of the object is in the post-ovulatory phase. For example, the predetermined range (K3) may be equal to greater than 16 mm, but less than 18 mm. The controller 130 may divide the menstrual cycle of the object into the postmenstrual phase, the late proliferative near ovulation phase, and the post-ovulatory phase, but the number of the division in the menstrual cycle is not limited thereto.

In addition, the controller 130 may identify whether at least one of a user confirmation command and a user modification command related to the identified menstrual cycle is input or not.

The controller 130 may request a confirmation to a user on the identified menstrual cycle, and when the user inputs the confirmation command via the input 160, the controller 130 may identify the identified menstrual cycle as a final menstrual cycle. In this case, the controller 130 may display the final menstrual cycle.

When the user modification command on the identified menstrual cycle is input, the controller 130 may modify the identified menstrual cycle, wherein the controller 130 may display the modified menstrual cycle.

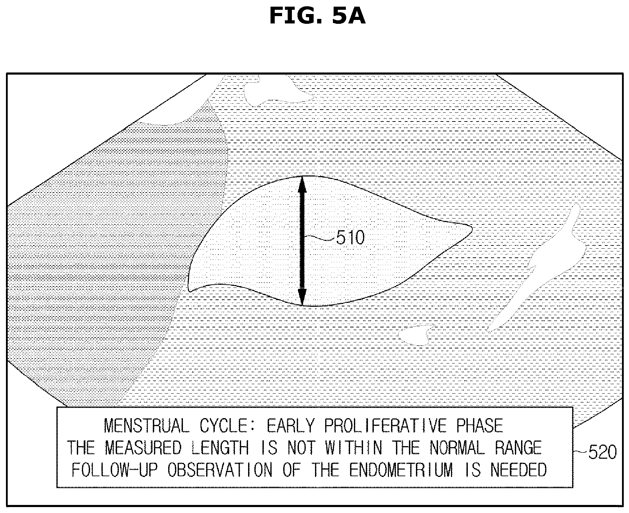

FIGS. 5A to 5C are views illustrating an example of a notification screen displayed on the ultrasound imaging apparatus according to an embodiment.

Referring to FIG. 5A, the controller 130 may measure the diagnostic parameters for the uterus of the object by using an ultrasonic echo signal.

For example, the controller 130 may measure the endometrial thickness using an ultrasonic echo signal. At this time, the controller 130 may automatically set a region for measuring the endometrial thickness, or may receive an input of a measurement region from the user through the input 160.

The controller 130 may identify whether the measured diagnostic parameter value is normal, based on the identified menstrual cycle.

Particularly, the controller 130 may identify whether the measured diagnostic parameter value is normal, by depending on whether the measured diagnostic parameter value is in a reference value range, wherein the range is predetermined according to the identified menstrual cycle. At this time, the reference value according to the identified menstrual cycle represents a theoretical value of a diagnostic parameter value for each menstrual cycle.

When the measured diagnostic parameter value is not in the reference value range related to the identified menstrual cycle, the controller 130 may identify that the diagnostic parameter value is not normal.

For example, the controller 130 may identify whether the measured endometrial thickness is normal, depending on whether the measured endometrial thickness is within the range of the reference value of the endometrial thickness related to the identified menstrual cycle.

When the measured endometrial thickness is not in the range of the reference value related to the identified menstrual cycle, the controller 130 may identify that the measured endometrial thickness is not normal.

When it is identified that the measured endometrial thickness is abnormal, the controller 130 may control the display 150 so that the display 150 provides a notification indicating that there is an abnormality in the endometrium, to a user.

The controller 130 may display a color marker 510 at a region, which is set to measure the endometrial thickness, wherein the controller 130 may display the color marker 510 to distinguish the case in which the endometrial thickness is not normal, from a case in which the endometrial thickness is normal.

For example, the controller 130 may display a green marker when the measured endometrial thickness is normal, and display a red marker when the measured endometrial thickness is not normal, thereby providing the notification indicating that there is an abnormality in the endometrium, to a user.

In addition, the controller 130 may control the display 150 so that the display 150 provides the notification indicating that there is an abnormality in the endometrium, to a user by displaying a separate notification window 520.

At this time, by displaying the identified menstrual cycle on the separate notification window 520, the controller 130 may provide information related to the menstrual cycle of the object, to the user while providing the notification indicating the presence of the abnormality in the endometrium.

The controller 130 may display the identified menstrual cycle by using characters, pictures, graphs, and colors, and the display method is not limited to the above-described method.

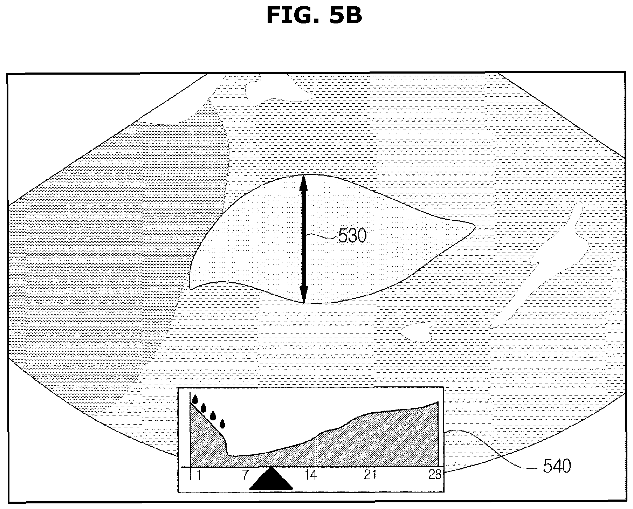

For example, the controller 130 may display the identified menstrual cycle using a separate graph 540, as illustrated in FIG. 5B.

The controller 130 may display the identified menstrual cycle, by using the graph 540, wherein the graph 540 indicates the change in the thickness of the uterine wall as time passes. Accordingly, the user can intuitively recognize the identified menstrual cycle.

The controller 130 may display the color marker 530 as described above together with the graph 540 indicating the thickness of the uterine wall, wherein the controller 130 may display the color marker 530 and the graph 540 to distinguish the case in which the endometrial thickness is not normal, from a case in which the endometrial thickness is normal.

The controller 130 may display the identified menstrual cycle through a separate interface, and may display information related to the menstrual cycle together with the measured diagnostic parameters.

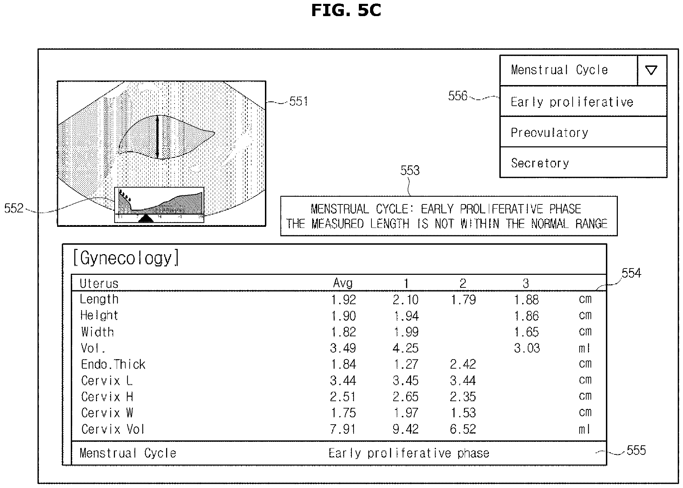

For example, as shown in FIG. 5 c, the controller 130 may display a table 554 indicating the measured diagnostic parameter with an ultrasound image 551 and simultaneously, display information related to the menstrual cycle.

The controller 130 may display the table 554 indicating the measured diagnostic parameter by adding a table 555 including information related to the identified menstrual cycle. The controller 130 may display information related to the menstrual cycle using a color, which is different from the color of the diagnostic parameter.

As described above, the controller 130 may display information related to the identified menstrual cycle by using at least one of the table 555, the graph 552, and the notification window 553 including information related to the menstrual cycle.

The controller 130 may request the user to confirm the displayed menstrual cycle, and the user may modify the menstrual cycle through the separate interface 556. When the modification command for the menstrual cycle is input from the user, the controller 130 may display the modified menstrual cycle. At this time, the controller 130 may display the modified menstrual cycle by making a separate indication indicating that it is modified by the user.

When displaying the measured diagnostic parameters, the controller 130 may display the different colors to distinguish the case in which the measured diagnostic parameters are identified to be normal from the cases in which the measured diagnostic parameters are identified to be abnormal.

In addition, the controller 130 may display the measured menstrual cycle together with the information related to the measured diagnostic parameters, while displaying a predetermined range, which is used to identify the menstrual cycle, i.e., a reference value for the identified menstrual cycle.

Accordingly, the user can easily compare the measured value for the object with the theoretical value, and thus the convenience and accuracy of the diagnosis can be increased.

According to another embodiment, an ultrasound imaging apparatus 100 may receive at least one piece of information related to whether an object has a birth experience, and information related to whether an object experiences menopause, from the user, and may identify whether the size of the uterus of the object is normal, based on the received information.

The size of the uterus may vary according to whether an object has a birth experience, and generally, the size of the uterus in the case in which the object has a birth experience, may be greater than the size of the uterus in the case in which the object don't have a birth experience.

In addition, the size of the uterus may vary according to whether an object experiences menopause, and generally, the size of the uterus before the menopause, may be greater than the size of the uterus after the menopause.

The controller 130 may identify whether the size of the uterus is normal, by identifying whether the size of the uterus is within a predetermined range corresponding to at least one piece of information related to whether an object has a birth experience, and information related to whether an object experiences menopause, wherein the information is input from the user.

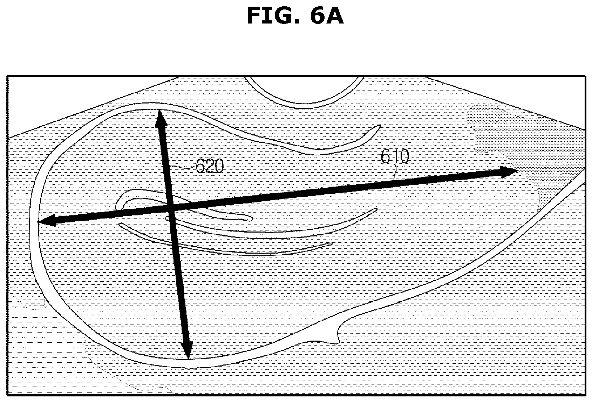

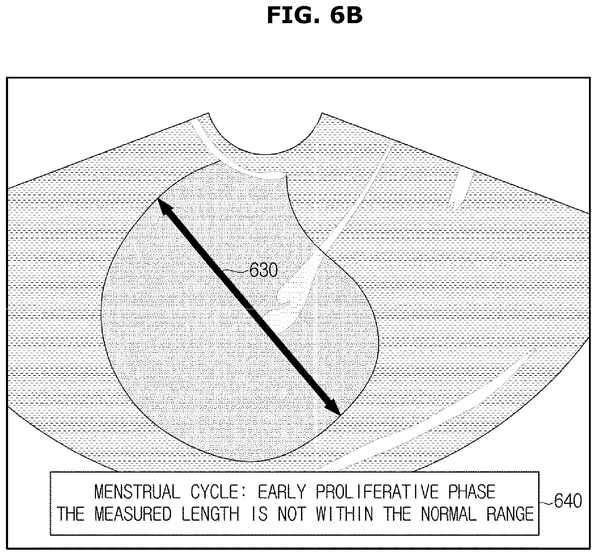

Hereinafter an operation of the controller 130 for measuring the size of the uterus and for identifying whether the measured size of the uterus is normal, will be described in detail with reference to FIGS. 6A and 6B.

FIGS. 6A and 6B are views illustrating an operation of measuring the size of the uterus and identifying whether the measured size of the uterus is normal, by the ultrasound imaging apparatus according to an embodiment.

According to an embodiment, the controller 130 may measure the size of the uterus on the ultrasound image. Particularly, the controller 130 may measure the size of the uterus by measuring at least one of width, height, and diagonal length of the uterus.

The controller 130 may identify whether the measured size of the uterus is normal, by identifying whether the size of the uterus is within a predetermined range corresponding to at least one piece of information related to whether an object has a birth experience, and information related to whether an object experiences menopause, wherein the information is input from the user.

When the measured size of the uterus is within the predetermined range corresponding to at least one piece of information related to whether an object has a birth experience, and information related to whether an object experiences menopause, wherein the information is input from the user, the controller 130 may identify that the measured size of the uterus is normal.

For example, referring to FIG. 6A, the controller 130 may measure the size of the uterus by measuring the width and height of the uterus.

When the object has a birth experience, the input 160 may receive a fact that the object has a birth experience, from the user. The controller 130 may identify whether the measured width and height of the uterus is within a predetermined width and height range in response to the case in which the object has a birth experience.

When the measured width and height of the uterus is within the predetermined width and height range in response to the case in which the object has a birth experience, the controller 130 may identify that the measured width and height of the uterus is normal, respectively.

The controller 130 may display markers 610 and 620 corresponding to the measured width and height of the uterus, and may display the width and height of the uterus using color markers that are distinguished from abnormal cases.

For another example, referring to FIG. 6B, the controller 130 may measure the size of the uterus by measuring the diagonal length of the uterus, wherein the diagonal length of the uterus may be measured based on ultrasound image of the transverse plane of the uterus.

When the object is in the postmenopausal state, the input 160 may receive the fact that the object is in the postmenopausal state, from the user. The controller 130 may identify whether the measured diagonal length of the uterus is within a predetermined diagonal length range in response to the case in which the object is in the postmenopausal state.

When it is identified that the measured diagonal length of the uterus is not within the predetermined diagonal length range in response to the case in which the object is in the postmenopausal state, the controller 130 may identify that the measured diagonal length of the uterus corresponding to the size of the uterus is not normal.

The controller 130 may display the size of the uterus by using a marker 630 indicating the diagonal length of the uterus, wherein the controller 130 may display the marker 630 in a color that is distinguished from the case in which the measured size of the uterus is normal.

The controller 130 may control the display 150 so that the display 150 displays that the measured diagonal length of the uterus is not in a normal range, by using a separate interface 640, and the controller 130 may display the display 150 so that the display 150 may display the above mentioned identified menstrual cycle.

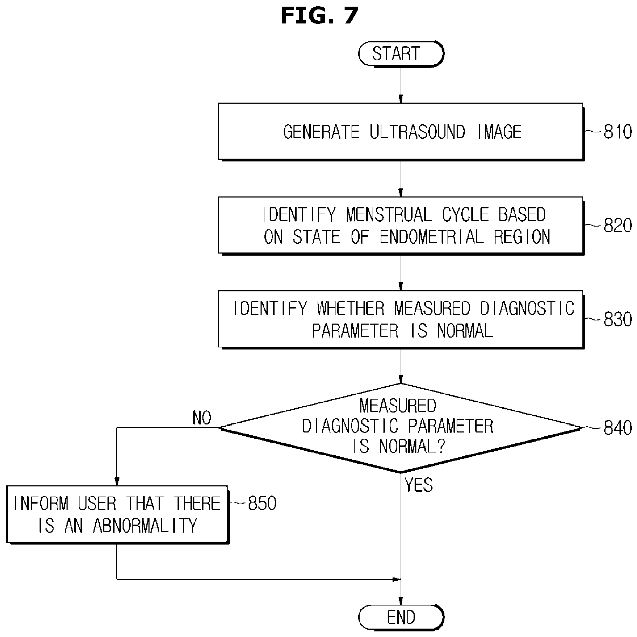

FIG. 7 is a flowchart illustrating a control method of the ultrasound imaging apparatus.

The ultrasound imaging apparatus 100 generates an ultrasound image (810), and identifies a menstrual cycle based on a state of an endometrial region on the generated ultrasound image (820).

In an embodiment outside the scope of the claims, the ultrasound imaging apparatus 100 may identify the menstrual cycle based on at least one of the brightness and the contrast of the endometrial region. Particularly, the ultrasound imaging apparatus 100 may identify the menstrual cycle based on at least one of the thickness of the endometrial region, the presence of the line having the high shade, and the number of the line on the ultrasound image.

In accordance with the invention, the ultrasound imaging apparatus 100 compares the image of the endometrial region with at least one of a pre-stored reference image and a previously measured image, and the ultrasound imaging apparatus 100 identifies a menstrual cycle corresponding to the image having a high matching rate, as a menstrual cycle of the object.

When the menstrual cycle of the object is identified, the ultrasound imaging apparatus 100 may identify whether the measured diagnostic parameter is normal or not, based on a predetermined range corresponding to the identified menstrual cycle (830, 840). Particularly, when the measured diagnostic parameter is within the predetermined range corresponding to the identified menstrual cycle, the ultrasound imaging apparatus 100 may identify that the measured diagnostic parameter is normal.

In this case, the diagnostic parameter represents a measurement value required for diagnosis, i.e., a measurement value for the diagnosis of the uterus, wherein the measurement value may include a length, a height, a width, an area and a volume of the uterus.

When the measured diagnostic parameter is not normal (NO in 840), the ultrasound imaging apparatus 100 may inform the user that there is an abnormality in the measured diagnostic parameter (850).

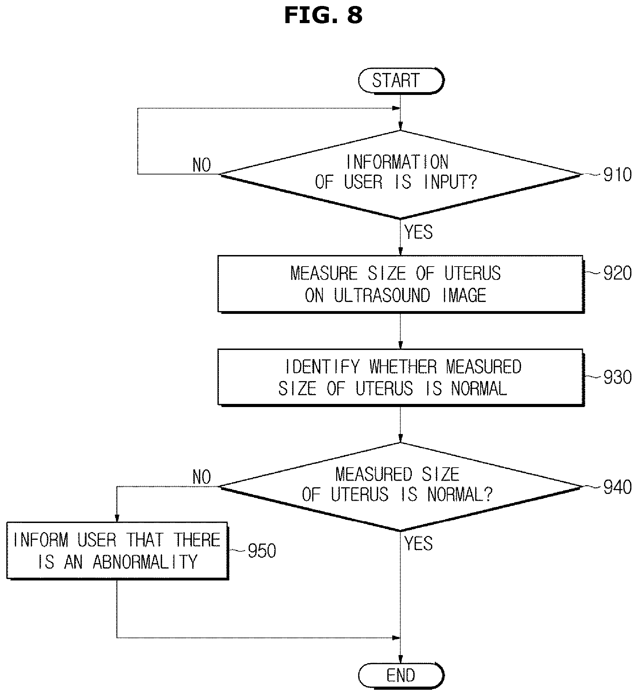

FIG. 8 is a flowchart illustrating a control method of the ultrasound imaging apparatus**.**

According an embodiment, the ultrasound imaging apparatus 100 may identify whether information of an object is input (910). The information of the object may include at least one piece of information related to whether an object has a birth experience, and information related to whether an object experiences menopause.

When at least one piece of information related to whether an object has a birth experience, and information related to whether an object experiences menopause is input, the ultrasound imaging apparatus 100 may measure the size of the uterus on the ultrasound image (920). Particularly, the ultrasound imaging apparatus 100 may measure the size of the uterus by measuring at least one of the width, height, and diagonal length of the uterus.

The ultrasound imaging apparatus 100 may identify whether the measured size of the uterus is normal (930, 940). Particularly, the ultrasound imaging apparatus 100 may identify whether the measured size of the uterus is normal, by depending on whether the measured size of the uterus is within a predetermined range corresponding to the information of the object.

When the measured size of the uterus is not within the predetermined range corresponding to the information of the object, the ultrasound imaging apparatus 100 may identify that the measured size of the uterus is not normal.

When the measured size of the uterus is not normal (No in 940), the ultrasound imaging apparatus 100 may provide a notification indicating that there is an abnormality in the size of the uterus, to the user (950).

Therefore, the ultrasound imaging apparatus 100 may provide more accurate diagnostic data and improve the accuracy and convenience of diagnosis.

As is apparent from the above description, it may be possible to improve the accuracy and convenience of diagnosis by providing information related to the menstrual cycle of the object, to a user upon the diagnosis of the uterus by using the ultrasound image.

Although a few embodiments of the present disclosure have been shown and described, it would be appreciated by those skilled in the art that changes may be made in these embodiments without departing from the scope of the disclosure which is defined in the claims.

## Claims

1. An ultrasound imaging apparatus (100) comprising:
a processor (140) configured to obtain an ultrasound image;
a display (150) configured to display the ultrasound image; and
a controller (130) configured to identify a menstrual cycle based on a state of an endometrial region on the ultrasound image and configured to control the display (150) to display the identified menstrual cycle;
wherein the controller (130) identifies the menstrual cycle based on a result of a comparison of the image of the endometrial region with a pre-stored image.

2. The ultrasound imaging apparatus (100) of claim 1, further comprising an input configured to receive at least one of a user confirmation command and a user modification command related to the identified menstrual cycle, wherein when the confirmation command is input, the controller (130) controls the display (150) to display the identified menstrual cycle and when the modification command is input, the controller (130) modifies the identified menstrual cycle and controls the display to display the modified menstrual cycle.

3. The ultrasound imaging apparatus (100) of any of the preceding claims, wherein the controller (130) measures a diagnostic parameter by using the ultrasound echo signal and identifies whether the measured diagnostic parameter is normal, based on the identified menstrual cycle;
wherein the diagnostic parameter comprises at least one of a length, a height, a width, an area, and a volume of the uterus.

4. The ultrasound imaging apparatus (100) of claim 3, wherein the controller (130) identifies that the diagnostic parameter is not normal when the measured diagnostic parameter is not within a range that is pre-determined according to the identified menstrual cycle.

5. The ultrasound imaging apparatus (100) of claim 3 or 4, wherein when the measured diagnostic parameter is not normal, the controller (130) controls the display (160) so that the display notifies the user that there is an abnormality.

6. The ultrasound imaging apparatus (100) of claim 5, wherein the controller (130) controls the display so that the display (160) notifies the user that there is an abnormality by using at least one of a color marker and a warning message.

7. The ultrasound imaging apparatus (100) of any of the preceding claims, further comprising: an input configured to receive at least one piece of information related to whether an object has a birth experience and information related to whether an object experiences menopause, wherein the controller (130) measures the size of the uterus by using the ultrasound echo signal, and identifies whether the measured size of the uterus is normal, based on the input information.

8. The ultrasound imaging apparatus (100) of claim 7, wherein when the measured size of the uterus is not within a reference range related to the input information, the controller (130) identifies that the measured size of the uterus is not normal and the controller (130) controls the display (160) so that the display (160) notifies the user that there is an abnormality.

9. A control method of an ultrasound imaging apparatus (100) comprising:
obtaining an ultrasound image;
displaying the ultrasound image;
identifying, by a controller of the ultrasound imaging apparatus, a menstrual cycle based on a state of an endometrial region on the ultrasound image; and displaying the identified menstrual cycle;
wherein the identification of the menstrual cycle comprises identifying the menstrual cycle based on a result of a comparison of the image of the endometrial region with a pre-stored image.

10. The control method of claim 9, further comprising: receiving at least one of a user confirmation command and a user modification command related to the identified menstrual cycle, wherein the display of the identified menstrual cycle comprises when the confirmation command is input, displaying the identified menstrual cycle and when the modification command is input, modifying the identified menstrual cycle and displaying the modified menstrual cycle.

## Patentansprüche

1. Ultraschallbildgebungsvorrichtung (100), die Folgendes umfasst:
einen Prozessor (140), der konfiguriert ist, um ein Ultraschallbild zu erhalten;
eine Anzeige (150), die konfiguriert ist, um das Ultraschallbild anzuzeigen; und
eine Steuerung (130), die konfiguriert ist, um einen Menstruationszyklus basierend auf einem Zustand einer Endometriumregion auf dem Ultraschallbild zu identifizieren, und die konfiguriert ist, um die Anzeige (150) so zu steuern, dass sie den identifizierten Menstruationszyklus anzeigt;
wobei die Steuerung (130) den Menstruationszyklus basierend auf einem Ergebnis eines Vergleichs des Bildes der Endometriumregion mit einem zuvor gespeicherten Bild identifiziert.

2. Ultraschallbildgebungsvorrichtung (100) nach Anspruch 1, die ferner eine Eingabe umfasst, die konfiguriert ist, um zumindest eines von einem Benutzerbestätigungsbefehl und einem Benutzermodifizierungsbefehl betreffend den identifizierten Menstruationszyklus zu empfangen, wobei, wenn der Bestätigungsbefehl eingegeben wird, die Steuerung (130) die Anzeige (150) so steuert, dass sie den identifizierten Menstruationszyklus anzeigt, und wenn der Modifizierungsbefehl eingegeben wird, die Steuerung (130) den identifizierten Menstruationszyklus modifiziert und die Anzeige so steuert, dass sie den modifizierten Menstruationszyklus anzeigt.

3. Ultraschallbildgebungsvorrichtung (100) nach einem der vorhergehenden Ansprüche, wobei die Steuerung (130) einen diagnostischen Parameter unter Verwendung des Ultraschallechosignals misst und basierend auf dem identifizierten Menstruationszyklus identifiziert, ob der gemessene diagnostische Parameter normal ist;
wobei der diagnostische Parameter zumindest eines von einer Länge, einer Höhe, einer Breite, einer Fläche und einem Volumen der Gebärmutter umfasst.

4. Ultraschallbildgebungsvorrichtung (100) nach Anspruch 3, wobei die Steuerung (130) identifiziert, dass der diagnostische Parameter nicht normal ist, wenn der gemessene diagnostische Parameter nicht innerhalb eines Bereichs liegt, der nach dem identifizierten Menstruationszyklus vorbestimmt ist.

5. Ultraschallbildgebungsvorrichtung (100) nach Anspruch 3 oder 4, wobei die Steuerung (130), wenn der gemessene diagnostische Parameter nicht normal ist, die Anzeige (160) so steuert, dass die Anzeige den Benutzer darüber informiert, dass eine Anomalie vorliegt.

6. Ultraschallbildgebungsvorrichtung (100) nach Anspruch 5, wobei die Steuerung (130) die Anzeige so steuert, dass die Anzeige (160) den Benutzer unter Verwendung zumindest eines von einem Farbmarker und einer Warnmeldung darüber informiert, dass eine Anomalie vorliegt.

7. Ultraschallbildgebungsvorrichtung (100) nach einem der vorhergehenden Ansprüche, die ferner Folgendes umfasst: eine Eingabe, die konfiguriert ist, um zumindest eine Information darüber zu empfangen, ob eine Patientin eine Geburtserfahrung hat, und Informationen darüber, ob eine Patientin die Menopause erlebt, wobei die Steuerung (130) die Größe der Gebärmutter unter Verwendung des Ultraschallechosignals misst und basierend auf den eingegebenen Informationen identifiziert, ob die gemessene Größe der Gebärmutter normal ist.

8. Ultraschallbildgebungsvorrichtung (100) nach Anspruch 7, wobei, wenn die gemessene Größe der Gebärmutter nicht innerhalb eines Referenzbereichs betreffend die eingegebenen Informationen liegt, die Steuerung (130) identifiziert, dass die gemessene Größe der Gebärmutter nicht normal ist, und die Steuerung (130) die Anzeige (160) so steuert, dass die Anzeige (160) den Benutzer darüber informiert, dass eine Anomalie vorliegt.

9. Steuerungsverfahren für eine Ultraschallbildgebungsvorrichtung (100), das Folgendes umfasst:
Erhalten eines Ultraschallbildes;
Anzeigen des Ultraschallbildes;
Identifizieren eines Menstruationszyklus durch eine Steuerung der Ultraschallbildgebungsvorrichtung basierend auf einem Zustand einer Endometriumregion auf dem Ultraschallbild; und
Anzeigen des identifizierten Menstruationszyklus;
wobei das Identifizieren des Menstruationszyklus das Identifizieren des Menstruationszyklus basierend auf einem Ergebnis eines Vergleichs des Bildes der Endometriumregion mit einem zuvor gespeicherten Bild umfasst.

10. Steuerungsverfahren nach Anspruch 9, das ferner Folgendes umfasst: Empfangen zumindest eines von einem Benutzerbestätigungsbefehl und einem Benutzermodifizierungsbefehl betreffend den identifizierten Menstruationszyklus, wobei das Anzeigen des identifizierten Menstruationszyklus Folgendes umfasst:, wenn der Bestätigungsbefehl eingegeben wird, Anzeigen des identifizierten Menstruationszyklus, und wenn der Modifizierungsbefehl eingegeben wird, Modifizieren des identifizierten Menstruationszyklus und Anzeigen des modifizierten Menstruationszyklus.

## Revendications

1. Appareil échographique (100) comprenant :
un processeur (140) conçu pour obtenir une image ultrasonore,
un écran (150) conçu pour afficher l'image échographique, et
un contrôleur (130) conçu pour identifier un cycle menstruel compte tenu d'un état d'une région endométriale présente sur l'image échographique et conçu pour commander l'écran (150) de façon qu'il affiche le cycle menstruel identifié ;
ledit contrôleur (130) identifiant le cycle menstruel compte tenu du résultat d'une comparaison de l'image de la région endométriale avec une image pré-enregistrée.

2. Appareil échographique (100) selon la revendication 1, comprenant en outre une entrée conçue pour recevoir une commande de confirmation d'utilisateur et/ou une commande de modification d'utilisateur relatives au cycle menstruel identifié, ledit contrôleur (130), lorsque la commande de confirmation est entrée, commandant l'écran (150) de façon qu'il affiche le cycle menstruel identifié et ledit contrôleur (130), lorsque la commande de modification est entrée, modifiant le cycle menstruel identifié et commandant l'écran de façon qu'il affiche le cycle menstruel modifié.

3. Appareil d'imagerie ultrasonore (100) selon l'une quelconque des revendications précédentes, dans lequel le contrôleur (130) mesure un paramètre de diagnostic au moyen du signal d'écho ultrasonore et identifie si le paramètre de diagnostic mesuré est normal, compte tenu du cycle menstruel identifié ;
ledit paramètre de diagnostic comprenant une longueur, une hauteur, une largeur, une superficie et/ou un volume de l'utérus.

4. Appareil échographique (100) selon la revendication 3, dans lequel le contrôleur (130) identifie que le paramètre de diagnostic n'est pas normal lorsque le paramètre de diagnostic mesuré ne se situe pas dans une plage qui est prédéterminée en fonction du cycle menstruel identifié.

5. Appareil échographique (100) selon la revendication 3 ou 4, dans lequel, lorsque le paramètre de diagnostic mesuré n'est pas normal, le contrôleur (130) commande l'écran (160) de façon qu'il avertisse l'utilisateur d'une anomalie.

6. Appareil échographique (100) selon la revendication 5, dans lequel le contrôleur (130) commande l'écran (160) de façon qu'il avertisse l'utilisateur d'une anomalie au moyen d'un marqueur de couleur et/ou d'un message d'avertissement.

7. Appareil d'imagerie ultrasonore (100) selon l'une quelconque des revendications précédentes, comprenant en outre : une entrée conçue pour recevoir au moins une information relative au fait qu'un objet a une expérience de naissance et une information relative au fait qu'un objet est en ménopause, le contrôleur (130) mesurant la taille de l'utérus au moyen du signal d'écho ultrasonore, et identifiant si la taille mesurée de l'utérus est normale, compte tenu des informations d'entrée.

8. Appareil échographique (100) selon la revendication 7, dans lequel, lorsque la taille mesurée de l'utérus n'est pas dans une plage de référence liée aux informations d'entrée, le contrôleur (130) identifie que la taille mesurée de l'utérus n'est pas normale et le contrôleur (130) commande l'écran (160) de façon que l'écran (160) avertisse l'utilisateur d'une anomalie.

9. Procédé de commande d'un appareil échographique (100) comprenant :
l'obtention d'une image échographique,
l'affichage de l'image échographique,
l'identification, par un contrôleur de l'appareil échographique, d'un cycle menstruel compte tenu d'un état d'une région endométriale présente sur l'image échographique, et
l'affichage du cycle menstruel identifié ;
ladite identification du cycle menstruel comprenant l'identification du cycle menstruel compte tenu du résultat d'une comparaison de l'image de la région endométriale avec une image pré-enregistrée.

10. Procédé de commande selon la revendication 9, comprenant en outre : la réception d'une commande de confirmation d'utilisateur et/ou d'une commande de modification d'utilisateur relatives au cycle menstruel identifié ; l'affichage du cycle menstruel identifié comprenant, lorsque la commande de confirmation est entrée, l'affichage du cycle menstruel identifié et, lorsque la commande de modification est entrée, la modification du cycle menstruel identifié et l'affichage du cycle menstruel modifié.
